# FASCICULE DE BREVET EUROPEEN

(11) **EP 0 835 652 B1**
(45) Date de publication et mention de la délivrance du brevet: **17.03.1999**
(21) Numéro de dépôt: 97402296.4
(22) Date de dépôt: 01.10.1997
(51) Int. Cl.: A61K 7/48

(54) **Utilisation d'au moins un glycol comme agent de solubilisation de la mélatonine dans l'eau et compositions les contenant**
Verwendung von mindestens einem Glykol zur Solubilisierung von Melatonin in Wasser und sie enthaltende Zusammensetzungen
Use of at least one glycol in the solubilisation of melatonin in water and compositions containing them

(30) Priorité: 10.10.1996 FR 9612387
(43) Date de publication de la demande: 15.04.1998
(73) Titulaire: L'OREAL, 75008 Paris (FR)
(72) Inventeur: Simon, Pascal, 94400 Vitry (FR); Bordeaux, Dominique, 91240 Saint Michel/S/Orge (FR); Gabnebien, Didier, 92320 Chatillon (FR)
(74) Mandataire: Tezier Herman, Béatrice

(56) Documents cités:
- EP-A- 0 438 856
- EP-A- 0 578 620
- WO-A-86/05093
- DATABASE WPI Week 8646 Derwent Publications Ltd., London, GB; AN 86-300704 XP002032177 "Cosmetic compsn. used to protect skin - comprises melatonin in soln. cream etc." & JP 61 221 104 A (SHISEIDO) , 1 octobre 1986
- DATABASE WPI Week 9304 Derwent Publications Ltd., London, GB; AN 93-033597 XP002032178 "Cosmetic compsn. used to protect skin - comprises melatonin in soln., cream etc." & JP 61 221 106 A (SHISEIDO) , 1 octobre 1986
- DATABASE WPI Week 8644 Derwent Publications Ltd., London, GB; AN 86-289026 XP002032179 "Hair tonic compsn. - contg. melatonin for reduced toxicity" & JP 61 212 512 A (SHISEIDO) , 20 septembre 1986

## Description

La présente invention a pour objet l'utilisation de glycols pour solubiliser la mélatonine ou ses analogues dans des compositions comprenant une phase aqueuse substantiellement exempte d'alcool. Elle concerne également de nouvelles compositions cosmétiques aqueuses exemptes d'alcool, comprenant de la mélatonine et au moins un glycol approprié comme agent de solubilisation.

La mélatonine, ou N-acétyl-5-méthoxytryptamine, surtout connue pour son activité sur le rythme circadien régulant la production d'hormones, est également décrite pour son activité antioxydante (Reiter R J, Verhandung der Deutschen Zoologischen Gesellschaft, 87 (2), 1994, 195-204; Reiter R J & al., Neuroendocrinoll Letter, 15 (1-3), 1993, 103-113; Reiter R J & al., J. Pineal Res., 18 (1), 1995, 1-11), en particulier son activité antiradicalaire (Reiter RJ & al., Brazilian Journal of Medical and Biological Research, 26 (22), 1993, 1141-1155). La plupart des études des propriétés antioxydantes de la mélatonine portent sur les phénomènes d'oxydation liés au vieillissement du cerveau (Poeggeler B & al., J. Pineal Res., 14 (4), 1993, 151-168; Cagnoli C M & al., J. Pineal Res., 18 (4), 1995, 222-226; Melchiorri D & al., FASEB J., 9 (12), 1995, 1205-1210; Sewerynek E & al., Neuroscience Letters, 195 (3), 1995, 203-205.

La mélatonine a également été décrite pour son utilisation en dermo-cosmétique pour améliorer l'apparence de la peau (JP-A-61-221104; WO-A-86/05093), ou pour protéger la peau contre l'effet d'une irradiation aux rayonnements U.V. (EP-A-438 856 ; E. Bangha & al., Dermatology 191, [2], 176, 1995). Il est préconisé d'employer la mélatonine à des concentrations comprises entre 10⁻⁴ et 10 % en poids par rapport au poids total de la composition.

Ainsi, la demande de brevet WO-A-86/05093 décrit une composition cosmétique comprenant de la mélatonine pour augmenter la sensibilité de la peau aux oestrogènes, en particulier pour le traitement de l'acné ou la prévention de la chute des cheveux. Les quantités de mélatonine préconisées dans cette demande sont comprises d'une manière générale entre 10⁻⁴ et plus de 1% en poids par rapport au poids total de la composition.

De même, la demande de brevet EP-A-438 856, concernant des compositions pour la protection de la peau contre les effets des irradiations aux rayonnements U.V., préconise des quantités de mélatonine comprises entre 1 et 10% en poids pour des compostions topiques, le seul exemple de ce type de composition étant un onguent anhydre comprenant 10% de mélatonine.

Pour avoir une bonne efficacité de la mélatonine appliquée par voie topique, il est essentiel qu'elle soit bien solubilisée dans son support. Or, il a été constaté que la mélatonine, aux doses préconisées dans l'état de la technique n'était soluble ni dans l'eau, ni dans les huiles utilisées en cosmétique. Ainsi, la mélatonine est insoluble à 1 % en poids dans l'eau, les huiles minérales (huile de vaseline), les esters gras (palmitate d'éthyl-2 hexyle, benzoate de dodécyle, néopentanoate d'octyl-2 dodécyle), les éthers d'alcool gras (alcool myristilique polyoxypropyléné, alcool stéarique polyoxypropyléné, palmitate d'éthyl-2 hexyl glycéryl éther) ou les alcools gras (octyldodécanol, alcool isostéarique, alcool hexyl-2 décylique-1).

Pour solubiliser la mélatonine ou ses analogues dans des compositions fluides telles que des crèmes, des gels ou des lotions, il est nécessaire d'employer de l'éthanol.

Ainsi, la demande de brevet WO-A-86/05093 préconise d'employer entre 10 et 30 % en poids d'éthanol.

De même, la demande de brevet JP-A-61-221104 décrit différentes solutions comprenant entre 10⁻⁴ % et 0,01 % en poids de mélatonine et entre 3 et 8 % en poids d'éthanol, le rapport pondéral mélatonine / éthanol étant compris entre 1/30000 et 1/500.

De même, la demande de brevet EP-A-578 620 décrit différentes compositions sous formes de gels comprenant entre 5 et 30 % d'éthanol, le rapport mélatonine / alcool étant compris entre 1/450 et 1/10.

En fait, pour des compositions telles que décrites dans la demande de brevet WO-A-86/05093, il est nécessaire d'incorporer plus de 35 % en poids d'éthanol pour obtenir une composition stable, sans recristallisation avec 0,5 % en poids de mélatonine. Plus la composition comprendra de mélatonine ou ses analogues, plus la quantité d'alcool nécessaire à l'obtention d'une composition stable sera importante.

Or, l'emploi de telles quantités d'alcool n'est pas approprié pour la réalisation de compositions cosmétiques puisqu'elles provoquent un dessèchement de la peau et des phénomènes d'irritation, d'échauffement et de rougeurs. Si de tels phénomènes peuvent être acceptés pour des compositions pharmaceutiques où l'on recherche un équilibre entre l'effet bénéfique du principe actif (ici la mélatonine) et ses effets secondaires et/ou ceux de la composition, il en va différemment pour des compositions cosmétiques qui doivent être exemptes de tout effet secondaire inacceptable par l'utilisateur.

La Demanderesse a maintenant démontré que des glycols appropriés permettent de solubiliser la mélatonine ou ses analogues dans des compositions aqueuses substantiellement exemptes d'éthanol. La possibilité d'utiliser les glycols pour solubiliser la mélatonine n'a jamais été décrite dans les demandes de brevet ci-dessus.

La présente invention concerne donc l'utilisation d'une quantité appropriée d'au moins un glycol pour solubiliser la mélatonine ou ses analogues dans des compositions comprenant une phase aqueuse substantiellement exempte d'éthanol.

Par quantité appropriée d'au moins un glycol, on entend selon l'invention une quantité suffisante pour permettre la solubilisation de la mélatonine ou ses analogues dans la phase aqueuse de la composition et l'obtention d'une composition stable, sans recristallisation de la mélatonine ou ses analogues.

Par composition stable, on entend de préférence selon l'invention une composition dans laquelle la mélatonine ou ses analogues ne cristallisent pas lorsqu'on la laisse reposer au moins 4 semaines à 4°C.

Bien entendu, la quantité de glycol appropriée pour solubiliser la mélatonine ou ses analogues dépendra de la quantité de mélatonine ou ses analogues employée dans la composition.

Cette quantité dépendra également de la teneur en eau de la composition comprenant la mélatonine ou ses analogues.

Il est apparu que le rapport pondéral melatonine/glycol approprié pour solubiliser la mélatonine ou ses analogues selon l'invention peut aller jusqu'à 1/5. Il est entendu que la limite inférieure de ce rapport dépendra du glycol employé, de son innocuité, de l'absence de problèmes d'irritation et de rougeurs lorsqu'ils sont employés à trop fortes doses.

Dans le cas d'une composition purement aqueuse, c'est à dire substantiellement exempte de phase grasse, comme une lotion ou un gel aqueux, le rapport pondéral mélatonine / glycol est de préférence compris entre 1/60 et 1/15.

Dans le cas d'une composition comprenant également une phase grasse, comme une émulsion de type huile dans eau (H/E) ou eau dans huile (E/H), le rapport pondéral mélatonine / glycol est de préférence compris entre 1/15 et 1/5.

Par composition substantiellement exempte d'éthanol, on entend selon la présente invention une composition qui ne comprend pas d'éthanol ou qui comprend des traces d'éthanol en quantité insuffisante pour solubiliser ou améliorer la solubilité de la mélatonine. D'une manière préférentielle, une composition substantiellement exempte d'éthanol ne comprend pas d'éthanol.

D'une manière avantageuse, les glycols utiles selon l'invention sont choisis parmi les hydrocarbures en C₃-C₁₀, linéaires ou ramifiés, saturés ou insaturés comprenant au moins deux fonctions hydroxyle libres, les polyalkylène glycols comprenant au moins 2 unités alkylène et les éthers de polyalkylène glycols.

Par hydrocarbures en C₃-C₁₀ linéaires ou ramifiés comprenant au moins deux fonctions hydroxyle libres, on entend en particulier selon l'invention les hydrocarbures comprenant une fonction hydroxyle primaire et au moins une deuxième fonction hydroxyle en β de la première. Il s'agit notamment d'alkylène glycols en C₃-C₁₀ linéaires ou ramifiés, saturés ou insaturés comme le propylène glycol ou le butylène glycol-1,3.

Lorsque l'hydrocarbure est insaturé, il peut comprendre une, deux ou plusieurs insaturations, comme l'isoprène glycol.

Par alkylène, on entend de préférence selon l'invention les groupes alkylènes en C₂-C₄ linéaires ou ramifiés, saturés ou insaturés. ll s'agit en particulier des polyéthylène glycols comprenant 6 à 40 unités éthylène glycol, ou du dipropylène glycol.

Par éther de polyalkylène glycol, on entend de préférence selon l'invention les monoethers d'alklkyles en C₁-C₄ linéaires ou ramifiés des polyalkylène glycols définis ci-dessus, par exemple le monoéthyl éther de diéthylène glycol (transcutol).

Parmi les analogues de la mélatonine, on peut notamment citer ses dérivés tels que la 5-méthoxytryptamine, le 5-méthoxytryptophane, le 5-méthoxytryptophol, l'acide 5-méthoxyindole-3-acétique et la 6-hydroxymélatonine. On peut également citer des agonistes mélatoninergiques, tels que ceux décrits dans les demandes de brevets WO-A-95/17405, EP-A-447 285, EP-A- 527 687, EP-A-530 087 et EP-A-591 057. Ces composés sont d'origine naturelle ou synthétique.

La composition peut comprendre de fortes teneurs en mélatonine ou ses analogues, jusqu'à 10 % en poids par rapport au poids total de la composition. La présente invention est particulièrement adaptée pour les compositions comprenant entre 10⁻⁴ % et 10 % en poids de mélatonine ou ses analogues, de préférence entre 0,1 % et 5 % en poids. Bien entendu, la présente invention concerne également des compositions comprenant moins de 10⁻⁴ % en poids de mélatonine ou ses analogues mais nécessitant l'utilisation d'un polyol approprié pour assurer sa solubilité dans la phase aqueuse.

La présente invention concerne également une composition topique substantiellement exempte d'éthanol comprenant de la mélatonine ou ses analogues dans une phase aqueuse et une quantité appropriée d'au moins un glycol pour solubiliser la mélatonine ou ses analogues, le rapport pondéral mélatonine / glycol étant compris entre 1/60 et 1/5.

De manière avantageuse, la composition selon l'invention a une viscosité comprise entre 0,5 et 8 Pa.s.

La composition topique dans laquelle se trouve la mélatonine ou ses analogues, est une composition cosmétique ou dermatologique et peut se présenter sous toutes les formes galéniques à usage topique normalement utilisées, et le support physiologiquement acceptable peut être constitué par tout support usuel pour une composition cosmétique ou dermatologique. La composition peut avoir la forme notamment de solution aqueuse ou suspension huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux, ou encore de microcapsules ou microparticules, ou de dispersions vésiculaires de type ionique et/ou non ionique. Ces compositions sont préparées selon les méthodes usuelles.

Les quantités des différents constituants des compositions sont celles classiquement utilisées dans les domaines considérés.

Ces compositions constituent notamment des crèmes de nettoyage, de protection, de traitement ou de soin pour le visage, pour les mains, pour les grands plis anatomiques ou pour le corps (par exemple crèmes de jour, crèmes de nuit, crèmes démaquillantes, crèmes de fond de teint, crèmes solaires), des fonds de teint fluides, des laits de démaquillage, des laits corporels de protection ou de soin, des laits solaires, des lotions, gels ou mousses pour le soin de la peau, comme des lotions de nettoyage, des lotions solaires, des lotions de bronzage artificiel, etc.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 à 30 % en poids, et de préférence de 0,5 à 20 % en poids par rapport au poids total de la composition. L'émulsion peut, en outre, contenir des vésicules lipidiques.

De façon connue, la composition cosmétique ou dermatologique peut contenir également des adjuvants habituels dans le domaine cosmétique ou dermatologique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans ces domaines, et par exemple de 0,01 à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles d'origine végétale (fraction liquide du beurre de karité, huile de tournesol), les huiles d'origine animale (perhydrosqualène), les huiles de synthèse (huile de Purcellin), les huiles ou cires de silicone (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose® 63 par la société Gattefosse.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium.

Dans la mesure où ils n'interférent pas avec l'activité de la mélatonine, les compositions selon l'invention peuvent contenir d'autres actifs destinés notamment à la prévention et/ou au traitement des affections cutanées.

Les compositions selon l'invention sont particulièrement appropriées pour prévenir ou traiter un stress oxydatif de la peau et/ou de ses annexes, en particulier lié aux U.V., au vieillissement, à l'inflammation, à l'alopécie, etc.

Les exemples ci-après permettent d'illustrer l'invention, sans toutefois chercher à en limiter la portée. Dans les compositions, les proportions indiquées sont des pourcentages en poids par rapport au poids total de la composition.

### Exemple 1: Etude de stabilité des compositions

La stabilité de la mélatonine dans différentes compositions a été étudiée.

### a) Stabilité dans une solution hydroalcoolique

La stabilité d'une solution hydroalcoolique de 0,5 % de mélatonine a été étudiée à 4°C pour différentes concentrations d'éthanol. Les résultats sont reportés sur le tableau ci-dessous.

| **Ethanol %** | **20 %** | **30 %** | **35 %** | **40 %** |
|---|---|---|---|---|
| **Stabilité 4 semaines** | non | non | oui | oui |
| **Stabilité 8 semaines** | non | non | non | oui |

Il faut donc au moins 35 % d'éthanol pour obtenir une composition stable de 0,5 % de mélatonine.

### b) Stabilité dans une composition hydroglycolique

La stabilité d'une solution hydroglycolique de 0,5 % de mélatonine a été étudiée à 4°C pour différentes concentrations d'isoprène glycol. Les résultats sont reportés sur le tableau ci-dessous.

| **Isoprène glycol %** | **20 %** | **30 %** | **50 %** |
|---|---|---|---|
| **Stabilité 4 semaines** | oui | oui | oui |
| **Stabilité 8 semaines** | oui | oui | oui |

L'utilisation d'isoprène glycol a permis d'obtenir des compositions a 0,5 % en poids de mélatonine stables jusqu'à 8 semaines à 4°C, pour des concentrations en glycol de 20 % dans l'eau.

De même, l'emploi d'isoprène glycol, de butylène glycol, de dipropylène glycol ou de polyéthylène glycol-6, permet d'obtenir des compositions à 2 % en poids de mélatonine, stables jusqu'à 8 semaines à 4°C, pour des concentrations en glycol de 50 % dans l'eau.

Dans des conditions similaires, l'emploi de dipropylène glycol ou de polyéthylène glycol-6, permet d'obtenir des compositions à 5 % en poids de mélatonine, stables jusqu'à 8 semaines à 4°C, pour des concentrations en glycol de 50 % dans l'eau.

### Exemple 2: Compositions selon l'invention

Les compositions a) à d) ci-dessous sont préparées selon les techniques usuelles. Elles se sont révélées stables au moins 4 semaines à 4°C. La mélatonine est compatible avec l'excipient.

### a) Lotion

| | |
|---|---|
| Butylène glycol | 20,00 % |
| Propylène glycol | 15,00 % |
| Acide citrique | 0,02 % |
| Citrate de sodium | 0,05 % |
| Mélatonine | 2,00 % |
| Parfum | 0,05 % |
| Conservateur | 0,10 % |
| Eau | qsp 100 |

La composition est stable. Aucune cristallisation n'a été observée après 4 semaines à 4 °C.

### b) Gel aqueux

| | |
|---|---|
| Isoprène glycol | 20,00 % |
| Glycérine | 5,00 % |
| Acrylate/C10C30 alkyl acrylate crosspolymère (Pemulen TR2 commercialisé par Goodrich) | 0,50 % |
| Alcool phényl éthylique | 0,50 % |
| Mélatonine | 0,50 % |
| Diazolidinyl uréa | 0,10 % |
| Eau | qsp 100 |

La composition est stable. Aucune cristallisation n'a été observée après 4 semaines à 4 °C.

### c) Emulsion E/H

| | |
|---|---|
| Diglycéryl di-isostéarate | 2,00 % |
| Laurylméthicone copolyol (Dow Corning Q-2-5200) | 2,00 % |
| Huile de vaseline | 10,00 % |
| Cyclométhicone | 15,00 % |
| PEG-4 | 15,00 % |
| Butylène glycol | 20,00 % |
| Mélatonine | 4,00 % |
| Sulfate de magnésium | 1,00 % |
| Méthyl paraben | 0,25% |
| Chlorphenesine | 0,30 % |
| Eau | qsp 100 |

La composition est stable. Aucune cristallisation n'a été observée après 4 semaines à 4 °C.

### d) Emulsion H/E

| | |
|---|---|
| Glycéryl stéarate | 1,00 % |
| Glycéyl stéarate PEG 100 | 2,00 % |
| Alcool behenylique | 2,50 % |
| Acide stéarique | 1,50 % |
| Cire d'abeille | 4,00 % |
| Caprique/caprylique triglycéride | 7,00 % |
| Hydrogenated polyisobutène (Parleam) | 12,00 % |
| Polyacrylamide / C13-14 isoparaffine / laureth 7 (Sepigel 305 commercialisé par Seppic) | 0,05 % |
| Isoprène glycol | 20,00 % |
| Mélatonine | 3,00 % |
| Chlorphenesine | 0,30 % |
| Méthyl paraben | 0,20 % |
| Eau | qsp 100 |

La composition est stable. Aucune cristallisation n'a été observée après 4 semaines à 4 °C.

### Exemple 3 (comparatif)

L'émulsion H/E décrite en page 7, lignes 5 à 20 de la demande de brevet EP-A-578 620, a été reproduite. Dans cette composition à 0,08 % en poids de mélatonine, le rapport mélatonine / glycol est de 1/100. Aucune solubilisation de la mélatonine n'est observée. Elle reste cristalline dans l'émulsion.

## Revendications

1. Utilisation d'une quantité appropriée d'au moins un glycol pour solubiliser la mélatonine, ses dérivés ou les agonistes mélatoninergiques, dans des compositions comprenant une phase aqueuse substantiellement exempte d'éthanol c-à-d qui ne comprend pas d'éthanol ou qui comprend des traces d'éthanol en quantité insuffisante pour solubiliser ou améliorer la solubilité de la mélatonine.

2. Utilisation selon la revendication 1, caractérisée en ce que le rapport pondéral mélatonine / glycol est inférieur ou égal à 1/5.

3. Utilisation selon l'une des revendications 1 ou 2, caractérisée en ce que la composition est substantiellement exempte de phase grasse.

4. Utilisation selon la revendication 3, caractérisée en ce que le rapport pondéral mélatonine / glycol est compris entre 1/60 et 1/15.

5. Utilisation selon l'une des revendications 1 ou 2, caractérisée en ce que la composition comprend également une phase grasse.

6. Utilisation selon la revendication 5, caractérisée en ce que le rapport pondéral mélatonine / glycol est compris entre 1/15 et 1/5.

7. Utilisation selon l'une des revendications 1 à 6, caractérisée en ce que le glycol est choisi parmi les hydrocarbures en C₃-C₁₀, linéaires ou ramifiés, saturés ou insaturés comprenant au moins deux fonctions hydroxyle libres, les polyalkylène glycols comprenant au moins 2 unités alkylène et les éthers de polyalkylène glycols.

8. Utilisation selon la revendication 7, caractérisée en ce que le glycol est choisi parmi les hydrocarbures comprenant une fonction hydroxyle primaire et au moins une deuxième fonction hydroxyle en β de la première.

9. Utilisation selon la revendication 8, caractérisée en ce que le glycol est choisi parmi les alkylène glycols en C₃-C₁₀ linéaires ou ramifiés, saturés ou insaturés.

10. Utilisation selon la revendication 9, caractérisée en ce que le glycol est choisi parmi le propylène glycol, le butylène glycol-1,3 ou l'isoprène glycol.

11. Utilisation selon la revendication 7, caractérisée en ce que le glycol est choisi parmi les polyéthylène glycols comprenant 6 à 40 unités éthylène glycol, ou le dipropylène glycol.

12. Utilisation selon la revendication 7, caractérisée en ce que le glycol est choisi parmi les monoéthers d'alkyles en C₁-C₄ linéaires ou ramifiés des polyalkylène glycols , en particulier le monoéthyl éther de diéthylène glycol.

13. Utilisation selon l'une des revendications 1 à 12, caractérisée en ce que la quantité de mélatonine, de ses dérivés ou des agonistes mélatoninergiques dans la composition est inférieure ou égale à 10 % en poids par rapport au poids total de la composition.

14. Utilisation selon la revendication 13, caractérisée en ce que la quantité de mélatonine dans la composition est comprise entre 10⁻⁴ % et 10 % en poids par rapport au poids total de la composition, de préférence comprise entre 0,1 % et 5 % en poids.

15. Composition topique substantiellement exempte d'éthanol c-à-d qui ne comprend pas d'éthanol ou qui comprend des traces d'éthanol en quantité insuffisante pour solubiliser ou améliorer la solubilité de la mélatonine comprenant de la mélatonine, ses dérivés ou les agonistes mélatoninergiques dans une phase aqueuse, caractérisée en ce qu'elle comprend une quantité appropriée d'au moins un glycol pour solubiliser la mélatonine, ses dérivés ou les agonistes mélatoninergiques, le rapport pondéral mélatonine glycol étant compris entre 1/60 et 1/5.

16. Composition selon la revendication 15, telle que définie dans les revendications 2 à 13.

## Claims

1. Use of an appropriate amount of at least one glycol to dissolve melatonin, its derivatives or melatoninergic agonists in compositions comprising a substantially ethanol-free aqueous phase, i.e. which does not comprise ethanol or which comprises traces of ethanol in an amount insufficient to dissolve or improve the solubility of melatonin.

2. Use according to Claim 1, characterized in that the melatonin/glycol ratio by weight is less than or equal to 1/5.

3. Use according to either of Claims 1 and 2, characterized in that the composition is substantially free of fatty phase.

4. Use according to Claim 3, characterized in that the melatonin/glycol ratio by weight is between 1/60 and 1/15.

5. Use according to either of Claims 1 and 2, characterized in that the composition also comprises a fatty phase.

6. Use according to Claim 5, characterized in that the melatonin/glycol ratio by weight is between 1/15 and 1/5.

7. Use according to one of Claims 1 to 6, characterized in that the glycol is chosen from saturated or unsaturated, linear or branched, C₃-C₁₀ hydrocarbons comprising at least two free hydroxyl functional groups, polyalkylene glycols comprising at least 2 alkylene units, and polyalkylene glycol ethers.

8. Use according to Claim 7, characterized in that the glycol is chosen from hydrocarbons comprising a primary hydroxyl functional group and at least one second hydroxyl functional group in the β position with respect to the first.

9. Use according to Claim 8, characterized in that the glycol is chosen from saturated or unsaturated, linear or branched, C₃-C₁₀ alkylene glycols.

10. Use according to Claim 9, characterized in that the glycol is chosen from propylene glycol, 1,3-butylene glycol or isoprene glycol.

11. Use according to Claim 7, characterized in that the glycol is chosen from polyethylene glycols comprising 6 to 40 ethylene glycol units, or dipropylene glycol.

12. Use according to Claim 7, characterized in that the glycol is chosen from linear or branched C₁-C₄ monoalkyl ethers of polyalkylene glycols, in particular diethylene glycol monoethyl ether.

13. Use according to one of Claims 1 to 12, characterized in that the amount of melatonin, its derivatives or melatoninergic agonists in the composition is less than or equal to 10% by weight with respect to the total weight of the composition.

14. Use according to Claim 13, characterized in that the amount of melatonin in the composition is between 10⁻⁴% and 10% by weight with respect to the total weight of the composition, preferably between 0.1% and 5% by weight.

15. Substantially ethanol-free topical composition, i.e. which does not comprise ethanol or which comprises traces of ethanol in an amount insufficient to dissolve or improve the solubility of melatonin, comprising melatonin, its derivatives or melatoninergic agonists in an aqueous phase, characterized in that it comprises an appropriate amount of at least one glycol in order to dissolve the melatonin, its derivatives or melatoninergic agonists, the melatonin/glycol ratio by weight being between 1/60 and 1/5.

16. Composition according to Claim 15, as defined in Claims 2 to 13.

## Patentansprüche

1. Verwendung einer geeigneten Menge mindestens eines Glykols zur Solubilisierung von Melatonin, Melatonin-Derivaten oder melatoninergen Agonisten in Zusammensetzungen, die eine wäßrige Phase enthalten, die im wesentlichen frei von Ethanol ist, das heißt die entweder kein Ethanol enthält oder die nur Spuren von Ethanol in einer Menge enthält, die nicht ausreicht, das Melatonin zu solubilisieren oder die Solubilisierbarkeit des Melatonins zu verbessern.

2. Verwendung nach Anspruch 1, dadurch gekennzeichnet, daß das Gewichtsverhältnis Melatonin/Glykol kleiner als oder gleich 1/5 ist.

3. Verwendung nach Anspruch 1 oder 2, dadurch gekennzeichnet, daß die Zusammensetzung im wesentlichen frei von einer Fettphase ist.

4. Verwendung nach Anspruch 3, dadurch gekennzeichnet, daß das Gewichtsverhältnis Melatonin/Glykol im Bereich von 1/60 bis 1/15 liegt.

5. Verwendung nach Anspruch 1 oder 2, dadurch gekennzeichnet, daß die Zusammensetzung auch eine Fettphase enthält.

6. Verwendung nach Anspruch 5, dadurch gekennzeichnet, daß das Gewichtsverhältnis Melatonin/Glykol im Bereich von 1/15 bis 1/5 liegt.

7. Verwendung nach einem der Ansprüche 1 bis 6, dadurch gekennzeichnet, daß das Glykol unter den geradkettigen oder verzweigten, gesättigten oder ungesättigten C₃₋₁₀-Kohlenwasserstoffen, die mindestens zwei freie Hydroxygruppen aufweisen, den Polyalkylenglykolen mit mindestens zwei Alkyleneinheiten und den Polyalkylenglykolethern ausgewählt wird.

8. Verwendung nach Anspruch 7, dadurch gekennzeichnet, daß das Glykol unter den Kohlenwasserstoffen ausgewählt wird, die eine primäre Hydroxygruppe und mindestens eine zweite Hydroxygruppe in β-Stellung zur ersten Hydroxygruppe aufweisen.

9. Verwendung nach Anspruch 8, dadurch gekennzeichnet, daß das Glykol unter den geradkettigen oder verzweigten, gesättigten oder ungesättigten C₃₋₁₀-Alkylenglykolen ausgewählt ist.

10. Verwendung nach Anspruch 9, dadurch gekennzeichnet, daß das Glykol unter Propylenglykol, 1,3-Butylenglykol und Isoprenglykol ausgewählt ist.

11. Verwendung nach Anspruch 7, dadurch gekennzeichnet, daß das Glykol unter den Polyethylenglykolen mit 6 bis 40 Ethylenglykol-Einheiten und Dipropylenglykol ausgewählt ist.

12. Verwendung nach Anspruch 7, dadurch gekennzeichnet, daß das Glykol unter den geradkettigen oder verzweigten Mono-C₁₋₄-Alkylethern der Polyalkylenglykole und insbesondere dem Diethylenglykolmonoethylether ausgewählt wird.

13. Verwendung nach einem der Ansprüche 1 bis 12, dadurch gekennzeichnet, daß der Mengenanteil des Melatonins, der Melatonin-Derivate oder der melatoninergen Agonisten in der Zusammensetzung kleiner als oder gleich 10 Gew.-%, bezogen auf das Gesamtgewicht der Zusammensetzung, ist.

14. Verwendung nach Anspruch 13, dadurch gekennzeichnet, daß der Mengenanteil des Melatonins in der Zusammensetzung im Bereich von 10⁻⁴ bis 10 Gew.-%, bezogen auf das Gesamtgewicht der Zusammensetzung, vorzugsweise im Bereich von 0,1 bis 5 Gew.-% liegt.

15. Zusammensetzung zur topischen Anwendung, die im wesentlichen frei von Ethanol ist, das heißt die kein Ethanol enthält oder die nur Spuren von Ethanol in einer Menge enthält, die nicht ausreicht, das Melatonin zu solubilisieren oder die Solubilisierbarkeit des Melatonins zu verbessern, und die in einer wäßrigen Phase Melatonin, Melatonin-Derivate oder melatoninerge Agonisten enthält, dadurch gekennzeichnet, daß sie eine zur Solubilisierung des Melatonins, der Melatonin-Derivate oder der melatoninergen Agonisten geeignete Menge mindestens eines Glykols enthält, wobei das Gewichtsverhältnis Melatonin/Glykol im Bereich von 1/60 bis 1/5 liegt.

16. Zusammensetzung nach Anspruch 15, die wie in den Ansprüchen 2 bis 13 definiert ist.
